Europäisches Patentamt

European Patent Office

Office européen des brevets

(11) Veröffentlichungsnummer: **0 280 972**
**A1**

## EUROPÄISCHE PATENTANMELDUNG

(21) Anmeldenummer: 88102452.5

(22) Anmeldetag: 19.02.88

(51) Int. Cl.⁴: **A61B 17/32** , A61B 17/39

(30) Priorität: 04.03.87 DE 3706968

(43) Veröffentlichungstag der Anmeldung:
07.09.88 Patentblatt 88/36

(84) Benannte Vertragsstaaten:
DE FR GB IT NL SE

(71) Anmelder: **Siemens Aktiengesellschaft Berlin
und München**
**Wittelsbacherplatz 2**
**D-8000 München 2(DE)**

(72) Erfinder: **Hagen, Uwe, Dipl.-Ing. (FH)**
**Auf der Hut 25**
**D-8550 Forchheim(DE)**

(54) **Handstück für eine Flüssigkeitsstrahl-Schneideinrichtung.**

(57) Das Handstück (8) umfaßt eine Düse (24), aus der ein Flüssigkeitsstrahl (42) unter hohem Druck über ein Endstück (8) austritt. Erfindungsgemäß ist das Endstück (8) als aktive Koagulationselektrode eines HF-Chirurgiegeräts (10) ausgebildet. Dabei kann es sich sowohl um eine monopolare als auch um eine bipolare Koagulationselektrode handeln. Der Vorteil für den Operateur besteht darin, daß er mit ein und demselben Handstück (8) bei der Operation sowohl schneiden als auch koagulieren kann.

FIG 1

EP 0 280 972 A1

## Handstück für eine Flüssigkeitsstrahl-Schneideinrichtung

Die Erfindung betrifft ein Handstück für eine Flüssigkeitsstrahl-Schneideinrichtung zum Schneiden in der Chirurgie mit einer Düse, aus der ein Flüssigkeitsstrahl unter hohem Druck über ein Endstück austritt.

Es sind Wasserstrahl-Schneidanlagen auf dem Markt (Firma AB Best Matic, Ronneby, Schweden; Prospekt "Der Spezialist für Wasserstrahl-Schneidanlagen"), mit denen Produkte aus Papier, Plastik, Gummi, Akustikmaterial, etc. mit sauberen Schnittflächen geschnitten werden können. Auch in der metallverarbeitenden Industrie werden solche Anlagen eingesetzt (Firma Press Cut AB, Ronneby, Schweden; Prospekt "Wasserschneiden", 1986). Das Prinzip des Schneidens mit Wasser besteht darin, einen Wasserdruck von maximal etwa 4000 kp/cm² (400 MPa) zu erzeugen und einen Wasserstrahl mit diesem Druck dann aus einer Düse mit einem Durchmesser von 0,1 bis 0,3 mm ausströmen zu lassen. Als Schneideflüssigkeit kommt hierbei reines Wasser zur Anwendung. Der Wasserstrahl wirkt wie ein Messer, was interessante Möglichkeiten für das Schneiden verschiedener Kurvenformen eröffnet.

Dieses Prinzip des Wasserstrahl-Schneidens wurde bereits auch auf dem medizinischen Sektor eingesetzt. Ein Handstück der eingangs genannten Art ist dabei aus der schwedischen Zeitschrift "Medicinsk Teknik", Nr. 5, Okt. 1986, S. 14 und 15, bekannt. Die dort vorgestellte Flüssigkeitsstrahl-Schneideinrichtung wurde bisher vorwiegend für Leberoperationen eingesetzt. Sie dürfte aber auch für chirurgische Eingriffe in andere parenchymatose Organe, wie in die Milz oder die Nieren, geeignet sein. Die Schneideinrichtung besteht aus einem druckerzeugenden Teil mit Ein-und Ausgängen für die Schneideflüssigkeit und einem Handstück mit auswechselbarer Düse und auswechselbarem Anschluß schlauch. Der Flüssigkeitsdruck kann stufenlos zwischen Null und 100 bar (10 MPa) variiert werden. Als Schneideflüssigkeit wird eine physiologische Lösung verwendet, z.B. eine Kochsalzlösung. Dieser können gerinnungshemmende oder heilungsfördernde Medikamente hinzugefügt werden. Der in der Chirurgie eingesetzte Flüssigkeits-Schneidestrahl hat einen Durchmesser von 80 μm und arbeitet mit einem Druck üblicherweise im Bereich von 30 bis 50 bar. Dadurch wird bei Leberoperationen das Leberparenchym zerstört; es entsteht ein etwas klaffender Schnitt, in dem die mittleren und großen Blutgefäße wie Brücken stehenbleiben. Diese können nach Bedarf koaguliert oder ligiert werden. Diejenigen Teile der Schneideinrichtung, die mit der Schneideflüssigkeit in Berührung kommen, sind aus einem säurefesten Edelstahl gefertigt.

Es hat sich herausgestellt, daß beim Flüssigkeitsstrahl-Schneiden ein zügiges Arbeiten möglich ist. Tritt jedoch eine Blutung im Gewebe auf, so wird in konventioneller Weise mit einem HF-Chirurgiegerät koaguliert. Dies bedeutet, daß bei einer Blutung das Handstück der Flüssigkeitsstrahl-Schneideinrichtung vom Operateur weggelegt werden muß, daß er die mit dem HF-Chirurgiegerät verbundene Koagulationselektrode in die Hand zu nehmen hat, und daß das HF-Chirurgiegerät sodann zwecks Koagulation einzuschalten ist. Dieses Vorgehen ist recht umständlich und verlangt eine unnötig lange Unterbrechung des Schneidvorganges.

Der Erfindung liegt die Aufgabe zugrunde, ein Handstück für einen Flüssigkeitsstrahl-Schneideinrichtung der eingangs genannten Art derart auszubilden, daß das umständliche Hantieren während der Operation beim Schneiden und Koagulieren wesentlich vereinfacht wird.

Diese Aufgabe wird erfindungsgemäß dadurch gelöst, daß das Endstück des Handstücks als aktive Koagulationselektrode eines HF-Chirurgiegeräts ausgebildet ist.

Hierdurch ergibt sich der erhebliche Vorteil, daß man mit ein und demselben Handstück bei der Operation sowohl schneiden als auch koagulieren kann.

Das Endstück des Handstücks, das in der Regel aus einem Metall wie einem Edelstahl gefertigt ist, kann dabei entweder als monopolare oder als bipolare Koagulationselektrode ausgebildet sein.

Weitere Ausgestaltungen der Erfindung sowie weitere Vorteile ergeben sich aus den Unteransprüchen und aus den beigefügten Figuren.

Im folgenden werden Ausführungsbeispiele der Erfindung anhand von sechs Figuren näher erläutert. Es zeigen:

Fig. 1 ein Handstück, dessen Endstück als monopolare Koagulationselektrode ausgebildet und sowohl an eine Flüssigkeitsstrahl-Schneideinrichtung als auch an ein HF-Chirurgiegerät angeschlossen ist;

Fig. 2 das Handstück nach Fig. 1 in vergrößerter Darstellung im Schnitt;

Fig. 3 ein weiteres Handstück, das sowohl an eine Flüssigkeitsstrahl-Schneideinrichtung als auch an ein HF-Chirurgiegerät angeschlossen ist, bei dem der Koagulationstrom durch einen Fußschalter geschaltet wird;

Fig. 4 ein Handstück für eine Flüssigkeitsstrahl-Schneideinrichtung, dessen Endstück gleichzeitig als bipolare Koagulationselektrode ausgebildet ist;

Fig. 5 die Koagulationselektrode nach Fig. 4 im Schnitt in vergrößerter Darstellung; und

Fig. 6 eine weitere Koagulationselektrode im Schnitt.

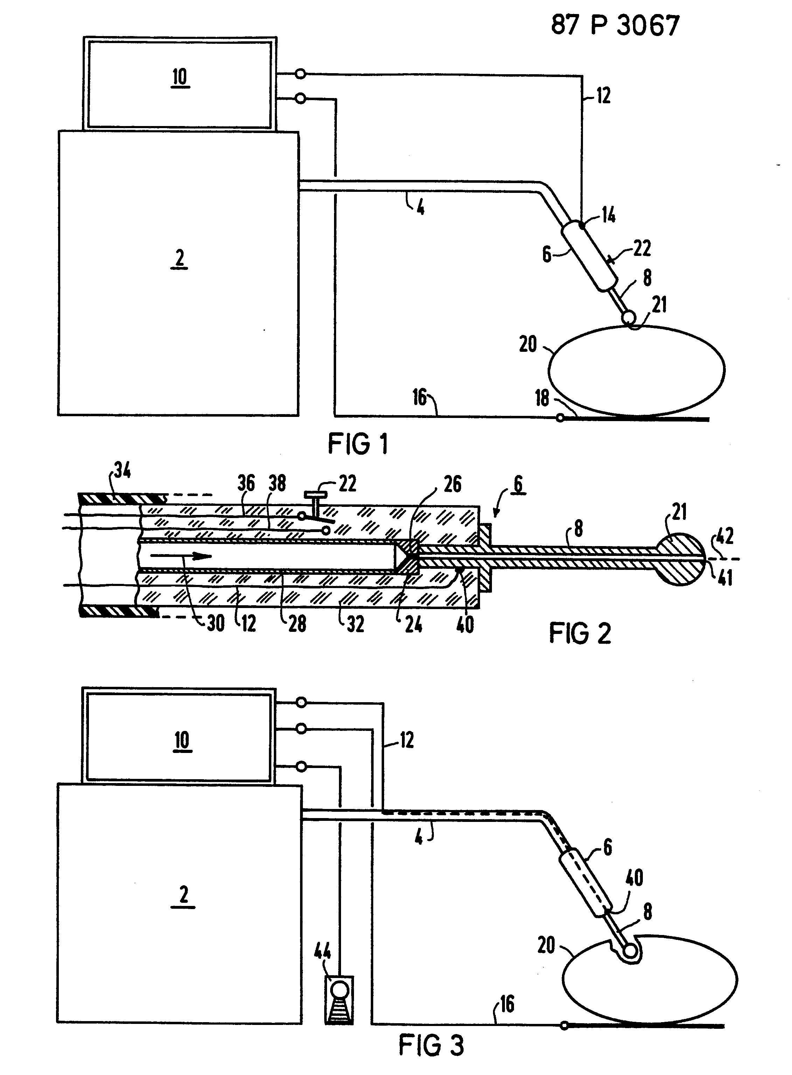

In Fig. 1 ist eine an sich bekannte Flüssigkeitsstrahl-Schneideinrichtung zum Schneiden in der Chirurgie ganz allgemein mit dem Bezugszeichen 2 versehen. Diese Schneideinrichtung 2 enthält in bekannter Weise ein Druckerzeugungssystem sowie ein Reservoir für die Schneideflüssigkeit, beispielsweise eine physiologische Kochsalzlösung. Das genannte Druckerzeugungssystem ist über einen Verbindungsschlauch 4, der die Schneidflüssigkeit unter hohem Druck führt, mit einem Handstück 6 für den Operateur verbunden. Dieser Handgriff 6 enthält eine (in Fig. 1 nicht gezeigte) Düse, aus der der Flüssigkeitsstrahl unter hohem Druck über ein längliches Endstück 8 austritt. Dieses Endstück 8 besteht aus einem Metall, also einem elektrisch leitfähigen Stoff. Das Endstück 8 ist speziell als aktive Koagulationselektrode zum Betrieb mit einem HF-Chirurgiegerät 10 ausgebildet. In Fig. 1 hat das Endstück 8 die Funktion einer monopolaren Koagulationselektrode.

Um im Operationsbetrieb mit dem Handgriff 6 nicht nur zu schneiden, sondern auch zu koagulieren, ist die HF-Koagulationsleitung 12 des HF-Chirurgiegeräts 10 mit einem Anschlußpunkt 14 am Handstück 6 verbunden, der wiederum mit dem Metall-Endstück 8 in elektrisch leitender Verbindung steht. Die Neutralenleitung 16 des HF-Chirurgiegeräts 10 ist an eine neutrale Elektrode 18 angeschlossen, die wiederum kontaktgebend am zu operierenden Patienten 20 anliegt. Der Patient 20 befindet sich während der Operation auf einer (nicht dargestellten) Patientenlagerungsplatte.

Wie aus Fig. 1 hervorgeht, ist das Endstück 8 - wie eine konventionelle Koagulations-Elektrode - bevorzugt am Ende kugelförmig ausgebildet. Im vorliegenden Fall enthält die Kugel 21 noch eine Auslaßöffnung für die Schneidflüssigkeit.

Ein wichtiges Requisit des in Fig. 1 dargestellten Handstücks 6 ist ein im vorderen Teil angebrachter Fingerschalter 22, wie er auf dem Gebiet der HF-Chirurgie zum Ein-und Ausschalten des Koagulationsstroms an sich üblich ist. Das in Fig. 1 gezeigte Düsen-Handstück 6 ermöglicht es also dem Operateur, durch Betätigung des Fingerschalters 22 beim Schneiden durchtrennte Blutgefäße zu koagulieren. Das Metall-Endstück 8 erfüllt somit zwei Funktionen. Zum einen dient es zum Führen des Flüssigkeitsstrahls beim Schneiden, und zum anderen dient es zum Verschließen blutender Gefäße.

In Fig. 2 ist in vergrößerter Darstellung ein Längsschnitt durch den Handgriff 6 gezeigt. Hier ist deutlich zu erkennen, daß das aus Metall bestehende, in seiner Längsrichtung durchbohrte Endstück 8 aus Metall an seinem äußeren Ende die Form einer Kugel 21 besitzt. Vor dem Endstück 8 ist die Düse 24 mit feiner Düsenöffnung 26 angeordnet. Der Düse 24 wird über eine Leitung 28 die unter hohem Druck stehende Schneidflüssigkeit zugeleitet, was durch einen Pfeil 30 angedeutet ist. Die Leitung 28 ist von einem Tragkörper 32 umgeben, welcher wiederum von einem leicht abwaschbaren Kunststoff-Mantel 34, der nur teilweise angedeutet ist, umschlossen ist. Der Fingerschalter 22 dient hier zum Einschalten des HF-Chirurgiegeräts 10 über zwei Verbindungsleitungen 36, 38. Wird der Fingerschalter 22 niedergedrückt, so wird der Kontakt geschlossen, die Verbindungsleitungen 36, 38 sind miteinander verbunden, und das HF-Chirurgiegerät 10 liefert einen Koagulationsstrom über die HF-Koagulationsleitung 12, die im vorliegenden Fall an einem Anschlußpunkt 40 direkt am Endstück 8 elektrisch leitend angeschlossen ist. Somit kann im Zustand "Koagulieren" der Koagulationsstrom über die HF-Koagulationsleitung 12, das Metall-Endstück 8 mit Kugel 21, den Patienten 20, die neutrale Elektrode 18 und die Neutralenleitung 16 zurück zum HF-Chirurgiegerät 10 fließen. Ist dagegen wieder der Zustand "Schneiden" gewünscht, wird der Fingerschalter 22 losgelassen und die Schneidflüssigkeit auf die Düse 24 gegeben. An der Auslaßöffnung 41 tritt dann der zum Schneiden verwendete Flüssigkeitsstrahl 42 aus.

In der Ausführungsform nach Fig. 3 sind am Handstück 6 keine Bedienelemente für das HF-Chirurgiegerät 10 angebracht. Dieses kann also eine glatte Oberfläche besitzen. Die Bedienung bei der Betriebsart "Koagulation" erfolgt hier über einen Fußschalter 44, wie er bei HF-Chirurgiegeräten im Stande der Technik üblich ist. Aber auch im vorliegenden Fall ist das Endstück 8 des Handstücks 6 als monopolare Koagulationselektrode ausgebildet. Im vorliegenden Fall ist weiter so vorgegangen, daß die HF-Koagulationsleitung 12 im Verbindungsschlauch 4 untergebracht ist. Dies ist gestrichelt angedeutet. Im übrigen entspricht die Ausführung nach Fig. 3 derjenigen nach Fig. 1 und 2.

Es soll noch angemerkt werden, daß das Endstück 8 am Handgriff 6 austauschbar befestigt sein kann. In diesem Fall sollte es, sobald es in den Handgriff 6 eingebracht ist, mit einem (nicht gezeigten) Verbindungsstück elektrisch verbindbar sein, an welches wiederum die HF-Koagulationsleitung 12 fest angeschlossen ist.

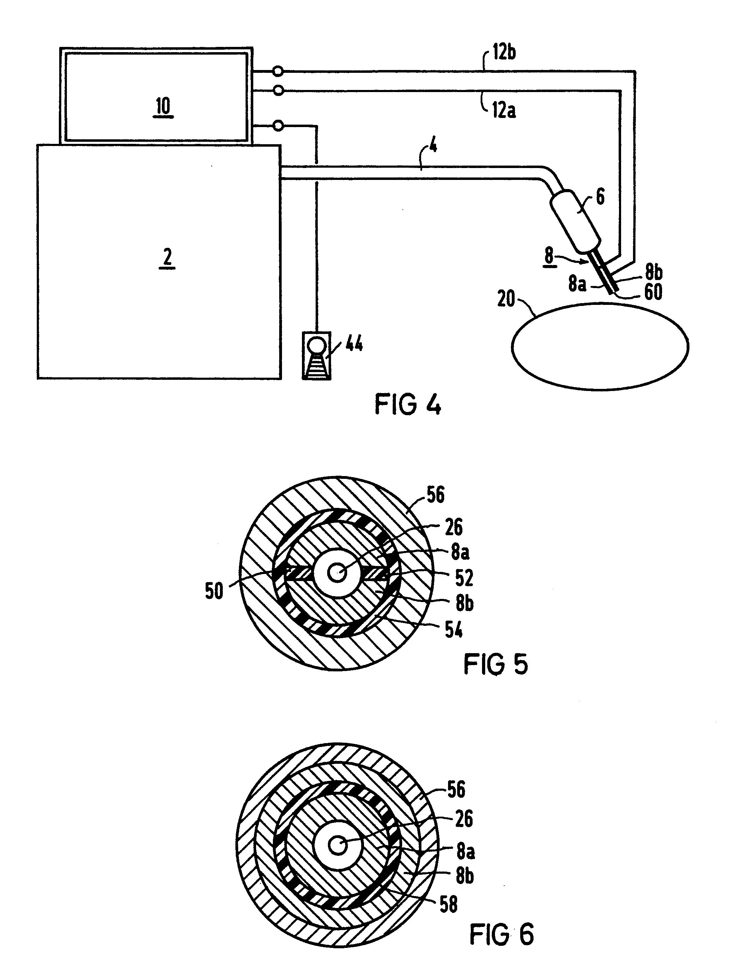

In den Figuren 4 bis 6 ist dargestellt, daß das Endstück 8 des Handstücks 6 auch als bipolare Koagulationselektrode ausgebildet sein kann. Für gleiche Bauelemente werden wiederum dieselben Bezugszeichen verwendet. Die aktive bipolare HF-Elektrode 8 besteht nach Fig. 4 aus zwei Elektroden, die im folgenden als erste und zweite bipolare Elektrode 8a, 8b bezeichnet werden. Diese beiden bipolaren Elektroden 8a, 8b sind elektrisch voneinander getrennt. Sie sind jeweils an eine Koagulations-Anschlußklemme des HF-Chirurgiegeräts 10 über Leitungen 12a bzw. 12b angeschlossen. Aus Fig. 4 geht hervor, daß lediglich die beiden Teile 8a, 8b des Endstücks 8 elektrisch leitend ausgebildet sind. Das restliche Handstück 6 kann aus Kunststoff bestehen.

In Fig. 5 ist eine Ausführungsform einer bipolaren Koagulationselektrode im Querschnitt gezeigt. Hier weist das Endstück 8 zwei halbschalenförmige Elektroden 8a, 8b auf, die durch isolierende, in Längsrichtung verlaufende Trennstücke 50, 52 voneinander getrennt sind. In Fig. 5 ist auch die Düsenöffnung 26 zu erkennen, die den für das Schneiden verwendeten Flüssigkeitsstrahl 42 im Durchmesser festlegt. Die beiden schalenförmigen Elektroden 8a, 8b sowie die isolierenden Trennstücke 50, 52 sind von einem Isolierschlauch 54 umgeben, der die Einzelteile zusammenhält. Dieser Isolierschlauch 54 wiederum kann von einer festeren Halterung 56 umgeben sein.

In Fig. 6 ist eine weitere bipolare Elektrode dargestellt, die eine konzentrische Anordnung aufweist. In diesem Beispiel ist die erste aktive bipolare Elektrode 8a ring-oder schlauchförmig ausgebildet. Sie ist konzentrisch zur Längsachse, in der die Düsenöffnung 26 liegt, angeordnet. Die erste oder innere Elektrode 8a ist von einem Isolierschlauch 58 eng umgeben, welcher wiederum von der zweiten ring-oder schlauchförmigen bipolaren Elektrode 8b umschlossen ist. Auch hier kann außen wiederum eine isolierende Halterung 56 angeordnet sein. Im vorliegenden Fall ist also die innere Elektrode 8a an die Zuleitung 12a und die äußere Elektrode 8b an die Zuleitung 12b des HF-Chirurgiegeräts 10 angeschlossen.

Bei den in den Figuren 4 bis 6 gezeigten aktiven bipolaren HF-Elektroden 8 wird beim Koagulationsvorgang das äußere Ende 60 auf die zu koagulierende Stelle gedrückt. Sodann fließt nach Betätigung des Fußschalters 44 über diese Stelle von der einen Elektrode 8a ein Koagulationsstrom zur anderen Elektrode 8b. Hierbei handelt es sich wiederum um einen HF-Strom vorgegebener Frequenz. Der Koagulationsstrom wird also vorliegend bevorzugt durch Betätigung des Fußschalters 44 ausgelöst. Auch bei dieser Ausführungsform kann der Operateur ohne großen Zeitverlust vom Schneiden zum Koagulieren, und umgekehrt, mit ein und demselben Handgriff 6 übergehen.

## Ansprüche

1. Handstück für eine Flüssigkeitsstrahl-Schneideinrichtung zum Schneiden in der Chirurgie mit einer Düse, aus der ein Flüssigkeitsstrahl unter hohem Druck über ein Endstück austritt, **dadurch gekennzeichnet,** daß das Endstück (8) als aktive Koagulationselektrode eines HF-Chirurgiegeräts (10) ausgebildet ist.

2. Handstück nach Anspruch 1, **dadurch gekennzeichnet,** daß das Endstück (8) als monopolare Koagulationselektrode ausgebildet ist (Fig. 1 bis 3).

3. Handstück nach Anspruch 2, **dadurch gekennzeichnet,** daß an das aus Metall bestehende Endstück (8) eine HF-Koagulationsleitung (12) angeschlossen ist.

4. Handstück nach Anspruch 2 oder 3, **dadurch gekennzeichnet,** daß das Endstück (8) an seinem äußeren Ende kugelförmig ausgebildet ist.

5. Handstück nach Anspruch 3 oder 4 mit einem Verbindungsschlauch zum Heranführen von Flüssigkeit zur Düse, **dadurch gekennzeichnet,** daß die HF-Koagulationsleitung (12) im Verbindungsschlauch (4) untergebracht ist.

6. Handstück nach einem der Ansprüche 2 bis 5, **gekennzeichnet durch** einen an ihm angebrachten Fingerschalter (22) zum Ein-und Ausschalten des Koagulationsstroms.

7. Handstück nach einem der Ansprüche 2 bis 5, **dadurch gekennzeichnet,** daß es keine Bedienelemente für das HF-Chirurgiegerät (10) aufweist.

8. Handstück nach einem der Ansprüche 3 bis 7, **dadurch gekennzeichnet,** daß das Endstück (8) austauschbar und mit einem Verbindungsstück elektrisch verbindbar ist, an welches die HF-Koagulationsleitung (12) fest angeschlossen ist.

9. Handstück nach Anspruch 1, **dadurch gekennzeichnet,** daß das Endstück (8) als bipolare Koagulationselektrode ausgebildet ist (Fig. 4 bis 6).

10. Handstück nach Anspruch 9, **dadurch gekennzeichnet,** daß das Endstück (8) zwei Elektroden (8a, 8b) aufweist, die durch eine Isolierung (50, 52; 58) voneinander getrennt sind, und daß an jede Elektrode (8a, 8b) eine Zuleitung (12a, 12b) angeschlossen ist.

11. Handstück nach Anspruch 9 oder 10, **dadurch gekennzeichnet,** daß die beiden Elektroden (8a, 8b) halbschalenförmig ausgebildet sind (Fig. 5).

12. Handstück nach Anspruch 9 oder 10, **dadurch gekennzeichnet,** daß das Endstück (8) zwei konzentrisch angeordnete Elektroden (8a, 8b) aufweist, von denen die innere Elektrode (8a) die Flüssigkeits-Austrittsöffnung (26) umschließt (Fig. 6).

87 P 3067

FIG 1

FIG 2

FIG 3

87 P 3067

FIG 4

FIG 5

FIG 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DD-A- 234 608 (VEB VOLKSWERFT) <br> * Insgesamt * | 1,2,6 | A 61 B 17/32 <br> A 61 B 17/39 |
| Y | | 3,4,7-12 | |
| | --- | | |
| Y | US-A-3 532 095 (MILLER) <br> * Spalte 1, Zeilen 4-14; Spalte 2, Zeilen 52-60; Zusammenfassung; Figuren * | 3,4,8 | |
| | --- | | |
| Y | FR-A-2 153 679 (LAMIDEX) <br> * Seite 1, Zeilen 14-30 * | 7 | |
| A | | 2,9 | |
| | --- | | |
| Y | DE-A-3 215 832 (U.S.D.E.) <br> * Seite 13, Zeilen 1-12; Seite 15, Zeilen 13-18; Figuren 2,8 * | 9-12 | |
| | --- | | |
| A | US-A-4 560 373 (SUGINO) <br> * Spalte 1, Zeile 45 - Spalte 2, Zeile 11; Spalte 3, Zeile 51 - Spalte 4, Zeile 17; Figuren * | 1 | |
| | --- | | **RECHERCHIERTE SACHGEBIETE (Int. Cl.4)** |
| A | DE-A-2 426 781 (DEMLING) <br> * Seite 3, Zeilen 10-13; Seite 4, Zeilen 6-8; Figuren 1,2 * | 5 | A 61 B |
| | --- | | |
| A | US-A-4 359 052 (STAUB) <br> * Zusammenfassung; Figur 3 * | 8 | |
| | --- | | |
| A | GB-A-2 165 761 (K.O.K.B.) <br> * Seite 1, Zeilen 122-128; Figur 2 * | 9-11 | |
| | --- | | |
| A | US-A-4 311 144 (HARADA) <br> * Spalte 2, Zeilen 20-26; Figuren * <br> ---        -/- | 9,10,12 | |

Der vorliegende Recherchenbericht wurde für alle Patentansprüche erstellt

| Recherchenort | Abschlußdatum der Recherche | Prüfer |
|---|---|---|
| DEN HAAG | 06-06-1988 | KLEIN C. |

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 085 658  (JACOBSEN) --- | | |
| A | GB-A-2 158 723  (K.N.I.I.O.N.K.) ----- | | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 06-06-1988 | KLEIN C. |